# EUROPEAN PATENT APPLICATION

(11) **EP 3 978 619 A1**
(43) Date of publication of application: **06.04.2022**
(21) Application number: 21187866.5
(22) Date of filing: 08.12.2016
(51) Int. Cl.: C12Q 1/68, G01N 33/53, G01N 33/58

(54) **AUTOMATED SILVER ENHANCEMENT SYSTEM**

(30) Priority: 09.12.2015 US 201562265114 P
(62) Divisional of application: 16873845.8
(71) Applicant: Intuitive Biosciences, Inc., Madison WI 53717 (US)
(72) Inventor: GUSE, Shawn, Madison, WI (US)
(74) Representative: Algemeen Octrooi- en Merkenbureau B.V.

(57) **Abstract**

The present invention provides automated systems and methods for enhanced silver staining of gold particles. The systems may preferably be used in an automated process for enhancing detection of gold-labelled probes on a microarray comprising discrete regions with a density of at least 20 of the discrete regions per cm², wherein silver ion solution and reducing agent solution are premixed prior to application to the solid support having the array thereon or in which the array is agitated after addition of the two solutions to the solid support having the array thereon.

## Description

### CROSS-REFERENCE TO RELATED APPLICATION

This application claims priority to and the benefit of U.S. Provisional Application No. 62/265,114, filed December 9, 2015, which is hereby incorporated by reference in its entirety.

### FIELD OF THE INVENTION

The present invention provides automated systems and methods for enhanced silver staining of gold particles.

### BACKGROUND OF THE INVENTION

A microarray is a multiplex lab-on-a-chip. It is a 2D array on a solid substrate (usually a glass slide or silicon thin-film cell) that assays large amounts of biological material using high-throughput screening miniaturized, multiplexed and parallel processing and detection methods. Types of microarrays include DNA microarrays, oligonucleotide microarrays, BAC microarrays, SNP microarrays, carbohydrate microarrays, protein microarrays, peptide microarrays, antibody microarrays, and non-natural ligand binder arrays.

In biological assays, microarrays are typically used with labeled probes. Typical labels include enzymes using colorimetric or luminescent substrates, organic fluorophores, fluorescent proteins, radioactive isotopes, and gold particles.

Accordingly, what is needed in the art are improved systems and methods for detecting binding interactions on a microarray.

### SUMMARY OF THE INVENTION

The present invention provides automated systems and methods for enhanced silver staining of gold particles. Current protocols and systems for detecting gold-labelled probes, especially on arrayed substrates, in automated systems have met with difficulty because of the sensitivity of the reagents and the relatively short working times. These difficulties are manifested in variability of staining across individual features in a microarray. The present invention addresses the problems by providing improved systems, methods and reagents for detecting gold-labelled probes.

In some embodiments, the present invention provides a system for automated detection of gold-labelled probes on a microarray comprising: a dispensing platform configured to receive a solid support having thereon an array comprising discrete regions with a density of at least 20 of the discrete regions per cm², each of the discrete regions being fixed with one species of capture molecule specific for an analyte, a plurality of reservoirs separately containing a plurality reagents comprising at least a silver ion solution, a reducing agent solution, one or more gold-labelled probes specific for the analyte, one or more test samples and one or more wash solutions, a reagent dispensing system coupled to the plurality of reservoirs and configured to dispense the plurality of reagents onto a solid support disposed on the dispensing platform, and a control system coupled with the dispensing system to allow ordered dispensation of the reagents onto a solid support disposed on the dispensing platform. In some embodiments, the system further comprise a mixing reservoir in fluid communication with the reservoirs contains the silver ion solution and the reducing agent solution. In some embodiments, the dispensing platform is configured for controlled agitation of the solid support. In some embodiments, the platform is heated. In some embodiments, the systems further comprise an optical detection system. In some embodiments, the optical detection system comprises an imaging device. In some embodiments, the imaging device is a CCD camera. In some embodiments, the dispensing platform, plurality of reservoirs, reagent dispensing system and control system are in a housing. In some embodiments, the dispensing platform is moveable between at least two positions and the movement is controlled by the control system. In some embodiments, the reagent dispensing system is moveable between at least two positions and the movement is controlled by the control system. In some embodiments, the silver ion solution comprises a solution of AgNO₃. In some embodiments, the molarity of the silver nitrate in the solution is from about 1 to about 50 mM. In some embodiments, the reducing agent solution comprising a reducing agent selected from the group consisting of p-aminophenol, phenidone (1-phenyl-3-pyrazolidinone) and hydroquinone. In some embodiments, the molarity of the reducing agent in the reducing agent solution is from about 0.5 to about 10 mM. In some embodiments, the solution further comprises sodium citrate at a concentration from about 50 to about 175 mM. In some embodiments, the solution further comprises citric acid at a concentration from about 50 to about 175 mM. In some embodiments, the pH of the reducing agent solution is from about 4.0 to about 5.0.

In some embodiments, the present invention provides an automated process for enhancing detection of gold-labelled probes on a microarray comprising: providing a solid support having thereon an array comprising discrete regions with a density of at least 20 of the discrete regions per cm², each of the discrete regions being fixed with one species of capture molecule bound to an analyte labelled with a gold probe, a first container comprising a silver ion solution and a second container comprising a reducing agent solution; dispensing the silver ion solution and the reducing agent solution into a third container; premixing the silver ion solution and the reducing agent solution to provide a premixed silver enhancement solution; dispensing the premixed silver enhancement solution onto the solid support; and incubating the solid support under conditions such that the silver ions nucleate on the gold particles, wherein the array comprises one or more discrete control regions in at least two separate quadrants on the array and the amount of silver precipitation at each of the discrete control regions varies by less than 10%. In some embodiments, the silver ion solution comprises a solution of AgNO₃. In some embodiments, the molarity of the silver nitrate in the solution is from about 1 to about 50 mM. In some embodiments, the reducing agent solution comprising a reducing agent selected from the group consisting of phenidone (1-phenyl-3-pyrazolidinone) and hydroquinone. In some embodiments, the molarity of the reducing agent in the reducing agent solution is from about 0.5 to about 10 mM. In some embodiments, the solution further comprises sodium citrate at a concentration from about 50 to about 175 mM. In some embodiments, the solution further comprises citric acid at a concentration from about 50 to about 175 mM. In some embodiments, the pH of the reducing agent solution is from about 4.0 to about 5.0. In some embodiments, the process is implemented by the automated system described above.

In some embodiments, the present invention provides an automated process for enhancing detection of gold-labelled probes on a microarray comprising: providing a solid support having thereon an array comprising discrete regions with a density of at least 20 of the discrete regions per cm², each of the discrete regions being fixed with one species of capture molecule bound to an analyte labelled with a gold probe, a first container comprising a silver ion solution and a second container comprising a reducing agent solution; dispensing the silver ion solution and the reducing agent solution onto the solid surface; agitating the solid support to mix the silver ion solution and the reducing agent solution; and incubating the solid support under conditions such that the silver ions nucleate on the gold particles, wherein the array comprises one or more discrete control regions in at least two separate quadrants on the array and the amount of silver precipitation at each of the discrete control regions varies by less than 10%. In some embodiments, the silver ion solution comprises a solution of AgNO₃. In some embodiments, the molarity of the silver nitrate in the solution is from about 1 to about 50 mM. In some embodiments, the reducing agent solution comprising a reducing agent selected from the group consisting of p-aminophenol, phenidone (1-phenyl-3-pyrazolidinone) and hydroquinone. In some embodiments, the molarity of the reducing agent in the reducing agent solution is from about 0.5 to about 10 mM. In some embodiments, the solution further comprises sodium citrate at a concentration from about 50 to about 175 mM. In some embodiments, the solution further comprises citric acid at a concentration from about 50 to about 175 mM. In some embodiments, the pH of the reducing agent solution is from about 4.0 to about 5.0. In some embodiments, the process is implemented by the automated system described above.

### DEFINITIONS

The terms "detecting" or "detection" or "determining the level" refer to quantitatively or non-quantitatively determination of the presence of the analyte(s) under investigation. "Detecting Formation of a Complex" refers to detecting a complex comprising a detector reagent by any method suitable for observing the particular label associated with the detector reagent.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include urine, cerebrospinal fluid, and blood products, such as plasma, serum and the like. Such examples are not however to be construed as limiting the sample types applicable to the present invention.

As used herein, the terms "protein," "polypeptide," and "peptide" refer to a molecule comprising amino acids joined via peptide bonds. In general "peptide" is used to refer to a sequence of 20 or less amino acids and "polypeptide" is used to refer to a sequence of greater than 20 amino acids. The proteins, polypeptides and peptides may be natural, produced by a recombinant process (i.e., expression of exogenous nucleic acid encoding the peptide, polypeptide or protein in an organism, host cell, or cell-free system) or produced by chemical synthesis.

The term "carrier" refers to a molecule that allows conjugation of additional, usually smaller, molecules such as peptides.

The term "specific binding partner (or binding partner)" refers to a member of a pair of molecules that interact by means of specific, noncovalent interactions that depend on the three-dimensional structures of the molecules involved. Typical pairs of specific binding partners include antigen/antibody, aptamer/target molecule, hapten/antibody, hormone/receptor, nucleic acid strand/complementary nucleic acid strand, substrate/enzyme, inhibitor/enzyme, carbohydrate/lectin, biotin/(strept)avidin, and virus/cellular receptor.

As used herein, the terms "immunoglobulin" or "antibody" refer to proteins that bind a specific antigen. Immunoglobulins include, but are not limited to, polyclonal, monoclonal, chimeric, and humanized antibodies, Fab fragments, F(ab')2 fragments, and includes immunoglobulins of the following classes: IgG, IgA, IgM, IgD, IbE, and secreted immunoglobulins (sIg). Immunoglobulins generally comprise two identical heavy chains and two light chains. However, the terms "antibody" and "immunoglobulin" also encompass single chain antibodies and two chain antibodies.

The phrase "specifically binds to an analyte" or "specifically immunoreactive with," when referring to an antibody, polypeptide, or aptamer, refers to a binding reaction which is determinative of the presence of the analyte (which can be an antibody or polypeptide) in the presence of a heterogeneous population of molecules such as proteins and other biologic molecules. Thus, under designated immunoassay conditions, the specified antibodies or polypeptides bind to a particular analyte and do not bind in a significant amount to other analytes present in the sample. A variety of immunoassay formats may be used to select antibodies or polypeptides specifically immunoreactive with a particular analyte. For example, solid-phase ELISA immunoassays are routinely used to select monoclonal antibodies polypeptides specifically immunoreactive with a protein or antibody See Harlow and Lane, Antibodies, A Laboratory Manual, CSHP, New York (1988), for a description of immunoassay formats and conditions that can be used to determine specific immunoreactivity.

The term "capture agent" refers to an unlabeled specific binding partner that is specific for (i) an analyte, or (ii) a detector reagent or an analyte, as in a competitive assay, or for (iii) an ancillary specific binding partner, which itself is specific for the analyte, as in an indirect assay. As used herein, an "ancillary specific binding partner" is a specific binding partner that binds to the specific binding partner of an analyte. For example, an ancillary specific binding partner may include an antibody specific for another antibody, for example, goat anti-human antibody.

A "capture area" as used herein is a region of a microarray or lateral flow device where the capture reagent is immobilized. A lateral flow device may have more than one capture area, for example, a "primary capture area," a "secondary capture area," and so on. Often a different capture reagent will be immobilized in the primary, secondary, or other capture areas. Multiple capture areas may have any orientation with respect to each other on the lateral flow substrate; for example, a primary capture area may be distal or proximal to a secondary (or other) capture area and vice versa. Alternatively, a primary capture area and a secondary (or other) capture area may be oriented perpendicularly to each other such that the two (or more) capture areas form a cross or a plus sign or other symbol.

The term "Western blot" refers to the analysis of protein(s) (or polypeptides) immobilized onto a support such as nitrocellulose or plastic membrane. The proteins are partitioned in acrylamide gels then transferred from the gel to a solid support, such as nitrocellulose or plastic membrane. The locations of the specific immobilized proteins are determined by exposure to antibodies with affinity to the antigen of interest. The binding of the antibodies may be detected by various methods, including the use of radiolabeled antibodies.

As used herein, the terms "protein microarray" and "protein chip" refer to protein-detecting molecules immobilized at high density on a substrate, and probed for various biochemical activities. (See, for example: Zhu H and Snyder M, "Protein chip technology", Current Opinion in Chemical Biology 7: 55-63, 2003; Cutler P, "Protein arrays: The current state of the art", Proteomics 3; 3-18, 2003; and MacBeath G, "Protein microarrays and proteomics", Nature Genetics Supplement 32: 526-532, 2002, each of which is incorporated herein by reference in its entirety).

"Blocking agent" means a molecular arrangement that will absorb to the surface of the substrate. The absorption can result because of non-covalent bonding attractive forces or because the blocking agent contains a reactive group. For example, a polyethylene glycol group can act as a blocking agent when it is covalently bonded to the surface or the protein bovine serum albumin can act as a blocking agent when it is non-covalently attached to the surface.

### DETAILED DESCRIPTION OF THE INVENTION

The present invention provides automated systems and methods for enhanced silver staining of gold particles. Current protocols and systems for detecting gold-labelled probes, especially on arrayed substrates, in automated systems have met with difficulty because of the sensitivity of the reagents and the relatively short working times. These difficulties are manifested in variability of staining across individual features in a microarray. The present invention addresses the problems by providing improved systems, methods and reagents for detecting gold-labelled probes.

### A. Automated Detection Systems, Processes and Reagents

Preferably, the automated systems comprise a housing containing a dispensing platform configured to receive a solid support having thereon an array (i.e., an "array chip") comprising discrete regions with a density of at least 20 features of the discrete regions per cm², each of the discrete regions being fixed with one species of capture molecule specific for an analyte, a plurality of reservoirs separately containing a plurality reagents comprising at least a silver ion solution, a reducing agent solution, one or more gold-labelled probes specific for the analyte, one or more test samples and one or more wash solutions, a reagent dispensing system coupled to the plurality of reservoirs and configured to dispense the plurality of reagents onto a solid support disposed on the dispensing platform, and a control system coupled with the dispensing system to allow ordered dispensation of the reagents onto a solid support disposed on the dispensing platform, and a reagent removal system that may comprise a vacuum. The systems may preferably to be used in an automated process for enhancing detection of gold-labelled probes on a microarray comprising wherein the silver ion solution and reducing agent solution are premixed prior to application to the solid support having the array thereon or in which the array is agitated after addition of the two solutions to the solid support having the array thereon.

The present invention is related to an automated method for identification and/or quantification of at least one target compound present in a biological sample by its binding upon a capture molecule fixed upon arrays of a solid support, the binding of the target compound upon its corresponding capture molecule resulting in the formation of a metal precipitate at the location of the capture molecule. In some embodiments, the present invention provides for improved automated detection of a target compound or analyte comprising the steps of: putting into contact the target compound with a capture molecule in order to allow a specific binding between the target compound with a (corresponding) capture molecule, the capture molecule being fixed upon a surface of a solid support according to an array comprising at least a density of 20 discrete regions per cm², each of the discrete regions being fixed with one species of capture molecules, using an automated system to perform a reaction, preferably a (chemical or biochemical) catalytic reaction, leading to a formation of a precipitate at the location of the binding, determining the possible presence of a precipitate in a discrete region preferably by the use of an optical detection system, and correlating the presence of the precipitate(s) at the discrete region(s) (precipitate pattern) with the identification and/or a quantification of the target compound in the biological sample.

The biological target compounds according to the invention may be present in a biological sample. The target compounds are preferably isolated purified, cleaved, copied and/or amplified if necessary by known methods by the person skilled in the art before their detection and/or quantification upon the array chips. Typical target compounds include specific proteins, viruses, hormones, drugs, nucleic acids and polysaccharides. In some embodiments, the proteins are specifically antibodies, e.g., IgD, IgG, IgM or IgA immunoglobulins, which bind to, for example, pathogenic organisms or tumors. Typical aqueous fluids include physiological fluids such as human or animal whole blood, blood serum, blood plasma, semen, tears, urine, sweat, saliva, cerebro-spinal fluid, vaginal secretions; in-vitro fluids used in research or environmental fluids such as aqueous liquids suspected of being contaminated by the analyte. When used with serum this may be used diluted or undiluted. Whole blood may also be used diluted but is preferably used diluted, e.g., in the range 1:1 to 1:10, but dilution up to about 1:5000 is still found to yield useful results. A suitable diluent is a standard specimen diluent, e.g., phosphate buffer saline.

The "array chips" according to the invention are any kind of solid support that allows the formation of arrays of capture molecules (specific pattern) upon one or more of its surfaces. A solid support according to the present invention is any support capable of binding a proteinaceous antigen, antibody, or nucleic acid molecules or other binding partners according to the present invention. Well-known supports, or carriers, include glass, silicon, polystyrene, polypropylene, polyethylene, polyvinylidene difluoride, dextran, nylon, natural and modified celluloses, and polyacrylamides. The nature of the carrier can be either soluble to some extent or insoluble for the purposes of the present invention. Those skilled in the art will know many other suitable solid support materials for binding biomolecules, or will be able to ascertain the same by use of routine experimentation.

Preferably, the arrays contain specific locations (advantageously presented according to a specific pattern), each of them containing normally only one species of capture molecule. Suitable capture molecules, include, but are not limited to, proteins including antigens and antigen binding proteins such as immunoglobulins, immunoglobulin fragments and single chain antibodies, and various nucleic acids including, but not limited to, single or double stranded deoxyribonucleic acid (DNA) and ribonucleic acid (RNA). In some embodiments, where the target compound or analyte is an immunoglobulin, for example an immunoglobulin suspected of being in the serum of a subject, the capture molecule is a protein or antigen which is bound by the target immunoglobulin. In some embodiments, where the target compound or analyte is a protein, the capture molecule is an antigen binding protein which binds the target protein. In some embodiments, where the target compound or analyte is a nucleic acid, the capture molecules is a nucleic acid which hybridizes to the target nucleic acid.

According to the invention, the specific locations on the array are smaller than 1000 µm in length. These locations or spots have preferably a diameter comprised between 10 and 500 µm and are separated by distance of similar order of magnitude, so that the array of the solid support comprises between 100 and 250,000 spots upon the surface of 1 cm². However, it is also possible to prepare spots smaller as 1 µm or less upon which the capture molecules are fixed. The formation of the spots or locations would be obtained by known microelectronic or photolitographic processes and devices that allow the fixation of the capture molecules on the surface of the solid support either by a covalent linkage or a non-covalent adsorption. The covalent linkage technique is preferred in order to control specifically the sites of capture molecules fixation and avoid possible drawbacks that may result with several capture molecules (like nucleic acids or antibodies) that can be desorbed during incubation or washing step.

In some embodiments, protein array chips utilize a solid support coated with ultrathin or clear nitrocellulose. See, e.g., US PAT PUBL. 20090253586 and 20090075828, both of which are incorporated herein by reference in their entirety. In preferred embodiments, the protein capture molecules are arrayed on the solid support. In multiplexed assays, a panel of capture reagents or antigenic compositions as described above is arrayed on the solid support. See, e.g., US PAT PUBL. 20090253586 and 20090075828, both of which are incorporated herein by reference in their entirety. Other suitable protein chip assays are described, for example, in US Patent 6,197,599; US Patent 6,294,790 and US Patent Application US20010014461A1, each of which is herein incorporated by reference in its entirety). In some embodiments, nucleic acid array chips are prepared by methods known in the art, for example by photolithographic techniques as described in the documents EP-0476014, U.S. Pat. Nos. 5,510,270, 5,445,934, WO97/29212, U.S. Pat. Nos. 5,605,662, 5,632,957 and WO94/22889. All these methods for the fixation of capture molecules on the surface of a solid support in order to obtain the above-described arrays are compatible with the present invention.

One of the preferred embodiments is the fixation of biological molecules like proteins, peptides or nucleic acid sequences by linkage of amino groups on activated glass bearing aldehyde moiety. The incorporation of an amine group in the nucleic acid chain is easily obtained using aminated nucleotide during their synthesis. Aminated amino acids can be fixed upon the surface of a solid support like glass bearing aldehyde groups as described by Schena et al. (Proc. Natl. Acad. Sci. USA, 93, pp. 10614 10619 (1996)) or as described in the document U.S. Pat. No. 5,605,662 and the publication of Krensky et al. (Nucleic Acids Research, 15, pp. 2891 2909 (1987)). The linkage between an amino and a carboxyl group is obtained by the presence of a coupling agent like carbodiimide compounds as described by Joos et al. (Anal. Biochem., 247, pp. 96 101 (1997)). Thiol modified oligonucleotides can be used also to obtain a reaction with amino groups upon the surface of a solid support in the presence of cross-linking molecules (Thrisey et al., Nucleic Acids Research, 24, pp. 3031 3039 (1996)). Similarly, oligonucleotides can be fixed to a gel like polyacrylamide bearing hydroxyl and aldehyde groups as described in the document U.S. Pat. No. 5,552,270 and WO98/28444.

A preferred embodiment of this invention is to take party of the amplification given by the catalytic reduction of Ag⁺ in the contact of other metals like gold. Gold nanoparticules are currently available and they can be easily fixed to molecules like protein. For example, streptavidin coated gold particles are available on the market and can conveniently be used in conjunction with biotinylated probes. Accordingly, in preferred embodiments, the present invention utilized detection probes that are labelled with gold nanoparticles. Suitable labelled probes include, but are not limited to, gold-labelled antigens, gold labelled Protein A or G, gold-labelled second antibodies (e.g., mouse, rabbit or goat gold-labelled anti-human IgG or IgM), gold-labelled biotin, gold-labelled strepaviden, and gold-labelled nucleic acids, or the same probes which are biotinylated and detectable with gold-labelled streptavidin or vice versa.

A preferred binding partner for these assays is an anti-immunoglobulin antibody ("second antibody") produced in a different species. Thus to detect a nonhuman primate antibody, a detectably labeled goat anti-simian immunoglobulin "second" antibody may be used. The solid phase support may then be washed with the buffer a second time to remove unbound antibody. The amount of bound label on the solid support may then be detected by conventional means appropriate to the type of label used (see below).

Such a "second antibody" may be specific for epitopes characteristic of a particular human immunoglobulin isotype, for example IgM, IgG₁, IgG₂ₐ, IgA and the like, thus permitting identification of the isotype or isotypes of antibodies in the sample which are specific for a given antigen. Alternatively, the second antibody may be specific for an idiotype of the target antibody in the of the sample.

As alternative binding partners for detection of the sample antibody, other known binding partners for human immunoglobulins may be used. Examples are the staphylococcal immunoglobulin binding proteins, the best know of which is protein A. Also intended is staphylococcal protein G, or a recombinant fusion protein between protein A and protein G. Protein G of group G and group C streptococci binds to the Fc portion of Ig molecules as well as to IgG Fab fragment at the V_{H3} domain. Protein C of Peptococcus magnus binds to the Fab region of the immunoglobulin molecule. Any other microbial immunoglobulin binding proteins, for example from Streptococci, are also intended (for example, Langone, J. J., Adv. Immunol 32:157 (1982)).

Thus, in some embodiments of the present invention utilize a labelled target molecule, which is then recognized by a conjugate. The labelled molecule can be considered as a first member of the binding pair. For DNA, the labelling is easily done by incorporation of biotinylated nucleotides during their amplification. For RNA, biotinylated nucleotides are used for their copy in cDNA or thereafter in the amplification step. Amplification of the nucleotide sequences is a common practice since the target molecules are often present in very low concentrations. Proteins are easily labelled using NHS-biotin or other reactions. Once the biotinylated molecules are captured, a streptavidin-gold complex, which is the second member of the binding pair, is added and the streptavidin specifically recognizes biotin, so that the complex is fixed at the location where the target is fixed. If haptens are used as label, an antibody-gold complex will be used. Direct labelling of the target molecules with gold is possible by using gold-labelled antigens, antibodies or nucleotides.

In preferred embodiments, the presence of the gold-labelled probes is detected by nucleation of a silver precipitate at the site of the gold-labelled probe. In some embodiments, a reactive mixture containing silver ions and a reducing agent is added to the surface and reduced silver will precipitate on the gold particles leading to the formation of significantly larger silver particles. The silver particles correspond to the location of the binding of biotinylated nucleotide sequence. As the location is well defined, it is possible to identify the presence of the silver particles (specific spot of the array). The precipitate has the form of small particles that reach with time a diameter of about 1 µm. The formation of these particles represents a real amplification of the signal since they originated from the presence of gold particles a few nm in diameter. The presence of the particles is preferably detected by an optical detector. The particles are preferred detected by illumination followed by detection of the amount of light that is transmitted through a surface. The presence of insoluble precipitate will absorb the light, and the amount of light which is transmitted through the surface is then detected and recorded. In other embodiments, the optical detector is a scanner which detects ad measures light reflected from the surface and which is not absorbed by the particles.In some embodiments, the optical detector can be a CCD or CMOS camera, which can measure a portion or overall of the array. Detection resolution is then dependent on the number of pixels of the camera. In other embodiments, the detector can be constituted of photo-diodes arranged into a line and the image is scanned by moving in front of this line.

Thus, in some preferred embodiments, the processes typically begins with an array chip comprising a plurality of biomolecule features (i.e., capture molecules). Multiplex assay chips may preferably comprise a number of different capture molecules at unique spots within the array on the array chip. The array chip is preferably then treating with a blocking agent (e.g., bovine serum albumin, fetal calf serum, gelatin, milk, etc.) to prevent nonspecific binding of sample and detection molecules. The blocked array is then incubated with a sample that may contain one or more target molecules then the surface is washed with a suitable buffer solution (preferably containing detergents, e.g., inonic or non-inonic surfactants) to remove non-binding materials. The gold nanoparticle labeled secondary binding molecule (typically an antibody) is added to the surface to specifically bind to the captured target molecule and, again, non-binding material removed with the buffered wash solution. Binding and washing can be facilitated with surface mixing, increased temperature, and macromolecular crowding agents. As described in more detail below, the bound gold nanoparticles on the surface are enhanced with the addition of silver deposition reagents, added sequentially or pre-mixed. The rate of the reaction is dependent on the concentration of reducing agent in the solution. When the reaction is complete, the surface is rinsed to remove excess silver deposition reagents, dried, and the surface imaged with a scanner or CCD camera. Software is used to quantify the transmission of light through or the reflection of light off of the surface in which the silver absorbs some of the light.

In preferred embodiments, these steps are automated and performed with a system for automated detection of gold-labelled probes on a microarray. In some embodiments, the systems comprise a dispensing platform configured to receive a solid support having thereon an array (i.e., an array chip as described above) comprising discrete regions with a density of at least 20 of the discrete regions per cm², each of the discrete regions being fixed with one species of capture molecule specific for an analyte. In some embodiments, the array chips are blocked prior to use in the automated system. In some preferred embodiments, the chips are glass or silicon and are encased in a single, dual, or multiple well plastic unit to create wells or reservoirs that surround the array.

In some embodiments, the systems comprise one or more dispensing platforms. In some embodiments, the dispensing platform is transportable between at least two positions and the movement is manipulated by the control system. In some embodiments, a plurality of dispensing platforms are arranged on a rotatable carousel configured to receive a plurality of array chips in a continuous series. The array chips are preferably delivered to the carousel from a hopper that is in mechanical communication with the carousel. In some embodiments, the dispensing platforms and/or carousel are heated so that the solid support can be heated to about 30 to 40 degrees C. In some embodiments, the dispensing platform is configured for controlled agitation of the solid support.

In further embodiments, the automated system comprises a plurality of reservoirs separately containing a plurality reagents selected from a silver ion solution, a reducing agent solution, one or more gold-labelled probes specific for the analyte, one or more test samples and one or more wash solutions, and a sample dilution solution. In some embodiments, the automated system comprises a reagent dispensing system coupled to the plurality of reservoirs and configured to dispense the plurality of reagents onto a solid support disposed on the dispensing platform. In some embodiments, the system further comprises a control system coupled with the dispensing system to allow ordered dispensation of the reagents onto a solid support disposed on the dispensing platform. In some embodiments, the systems further comprise a mixing reservoir in fluid communication with the reservoirs contains the silver ion solution and the reducing solution. In some embodiments, the reagent dispensing system is transportable between at least two positions and the movement is manipulated by the control system.

In preferred embodiments, the dispensing platform, plurality of reservoirs, reagent dispensing system and control system are provided in a housing.

Operation of the automated systems and methods of the present invention may be illustrated through use of a serum sample suspected of containing an analyte. The reagent dispensing system picks up sample dilution buffer followed by a serum sample and dispenses the sample into the reservoir on the array chip which is then agitated to mix the sample and allow immobilization of any target analyte present by the appropriate capture molecule in the array. The array chip is transferred to a heated dispensing platform on a rotatable carousel and warmed to approximately 10 to 40°C, preferably 35 to 40°C, and most preferably about 37°C for a specified time (e.g., 10-15 minutes) to capture the appropriate biomolecules from the sample. In some embodiments, the carousel is agitated during the incubation to improve the rate of binding. The array chip surface is washed with a continuous stream of wash solution that is simultaneously withdrawn by vacuum by the dispensing system.

The dispensing system then dispenses the gold-labelled probe (e.g., a gold labeled second antibody) solution onto the array chip, which is incubated for a specified time (e.g., about 10 to 15 minutes) on the heated carousel. The array chip is then washed and optionally rinsed with wash solution. The reducing agent solution and the silver ion solution are then premixed or delivered directed to the array chip via the dispensing system and the carousel is then agitated to mix the two solutions to provide a silver deposition reagent. After a specified time (from about 1 to 10 minutes, preferably 1 to 5 minutes, more preferably 2 to 4 minutes and most preferably about 3 minutes), the silver deposition reagent is removed by vacuum and rinsed with water (or rinse reagent). The array chip may then be optionally dried and then imaged with an imaging system as described in more detail below. In other embodiments, the surface is imaged without the reagent removing, washing and drying steps. A high resolution image is preferred to allow discrimination and quantification of features that have silver deposits, which is filtered in an algorithm to assign a value for each feature.

Accordingly, in some embodiments, the present invention provides an automated process for enhancing detection of gold-labelled probes on a microarray. In some embodiments, the methods comprise providing a solid support having thereon an array comprising discrete regions with a density of at least 20 of the discrete regions per cm², each of the discrete regions being fixed with one species of capture molecule bound to an analyte labelled with a gold probe, a first container comprising a silver ion solution and a second container comprising a reducing agent solution. In some embodiments, the methods further comprise dispensing the silver ion solution and the reducing agent solution into a third container and then premixing the silver ion solution and the reducing agent solution to provide a premixed silver enhancement solution. The premixed silver enhancement solution is then dispensed onto the solid support. The solid support may then be optionally agitated. In other embodiments, the silver ion solution and the reducing agent solution are dispensed onto the solid surface and the solid support is agitated to mix the silver ion solution and the reducing agent solution. The solid support is then incubated under conditions such that the silver ions nucleate on the gold probes.

In some embodiments, the amount of silver precipitate is related to the presence and/or amount of one or more target analytes in the samples. The amount of analyte may be determined from the degree of deposit, for example, by visual inspection, colorimetrically, photometrically, or by imaging (e.g., with a CCD or CMOS camera, including imaging with a smart phone). Use of standard curves or color charts for known analyte concentration absorbance values for the immobilized surface provides a convenient yet accurate measure of the amount of analyte in the test fluid. The invention is expected to be useful in the enhancement of all types of immunoassays in which colloidal gold is used as a labelling agent and other options will occur to a man skilled in the art of binding assays

In some preferred embodiments, the arrays on the array chips of the present invention comprise 2, 3, 4, 5, 6, 7, 8, 9, 10 or otherwise a plurality of control spots within the array. In some embodiments, the control spots comprise known amount of gold label, either the gold labeled probe of simply gold nanoparticles. In other embodiments, the control spots contain a known amount of an analyte, e.g., a positive control. In some embodiments, there may be multiple positive control spots for multiple analytes. The automated methods of the present invention allow for a high degree of reproducibility of signal (i.e., silver precipitation) between control spots located at different spots within the array. In this regard, a circular, square or rectangular array (indeed, an array of any shape) may be divided into a series of quadrants, each with a equal number of uniquely defined positions or spots. In some embodiments, the amount of silver precipitate formed at 2, 3, 4, 5, 6, 7, 8, 9, 10 or other a plurality of control spots within a corresponding number of quadrants with a known amount of gold, gold labeled probe or positive control analyte varies by less than 10%. For example, an array, ay be divided into 2, 3, 4, 5, 6, 7, 8, 9, or 10 or more quadrants, each with an equal number of spots. One or more control spots can preferably be included within each quadrant. The systems and methods of the present invention allow for less than 10% variability in silver precipitation at corresponding control spots (i.e., spots containing the same aount of gold, gold-labelled probe, or positive control analyte) within each of the quadrants.

As described above, the automated systems and processes of the present invention utilize two solutions to produce a silver precipitate in the presence of a gold label; a silver ion solution and a reducing solution. The solutions are preferably used in a ratio of about 1:1 and can be premixed before contact with the assay chips or added to the assay chips and then mixed. The ratio that is utilized is not important as long as the final concentrations in the mixed solutions are within the desired range. The silver ions utilized in the silver ion solution may be supplied by any ionizable silver compound provided that the counter ion does not otherwise interfere with the assay. Preferred silver compounds are salts of organic acids, especially silver nitrate, but any soluble inorganic silver salt, such as silver citrate or silver lactate, may be used. The concentration of silver ion in the solution may vary greatly but is preferably in the range 0.1 to 1000 mM, more preferably from 1 to 100 mM, particularly from 2 to 20 mM. Suitable agents for use in the reducing agent solution include, but are not limited to, an amino or hydroxybenzene such as hydroquinone, p-aminophenol, and phenidone (1-phenyl-3-pyrazolidinone)Suitable buffers include sodium maleate at a pH of about 7.5 with sodium chloride as an ionic stabilizer, as well as sodium citrate and sodium acetate buffer systems. The silver deposit may further be fixed using a suitable solution such as sodium thiosulfate although this is usually unnecessary. A suitable wash solution preferably comprises from about 20 to 70 mM sodium citrate, preferably 50 mM, from about 10 to 40 mM sodium HEPES, preferably 25 mM, pH 7.0 to 8.0, preferably 7.5, and from about 0.01% to 0.1% Tween 2, preferably about 0.05%.

In some particularly preferred embodiments, the silver ion solution utilized in the automated systems and processes of the present invention comprises an aqueous solution of AgNO₃. In some embodiments, the molarity of the silver nitrate in the solution is from about 1 to about 50 mM, preferably from about 5 to about 25 mM, and most preferably about 10 to 20 mM. In some further preferred embodiments, the reducing agent solution comprises p-aminophenol, phenidone (1-phenyl-3-pyrazolidinone) or hydroquinone. In some embodiments, the molarity of the reducing agent in the reducing agent solution is from about 0.5 to about 10 mM, preferably from about 1 to about 5 mM, more preferably from about 2 to about 4 mM and most preferably about 3 mM. In some embodiments, the reducing agent is provided in a citrate buffer. In some embodiments, the reducing agent solution comprises sodium citrate at a concentration from about 50 to about 175 mM, preferably from 80 to about 160 mM, and most preferably from about 130 to about 150 mM. In some embodiments, the reducing agent solution further comprises citric acid at a concentration from about 50 to about 175 mM, preferably from 80 to about 160 mM, and most preferably from about 130 to about 150 mM. In some embodiments, the pH of the reducing agent solution is from about 3.0 to 6.0, more preferably about, 4.0 to about 5.0, and most preferably about 4.4. In some embodiments, the reducing agent solution further comprises sodium thiosulfite at a concentration from about 0.05 to 5 mM, more preferably about 0.1 to 1 mM, and most preferably about 0.4 mM. In some embodiments, the reducing agent solution further comprises 4-hydroxy-D-phenylglycine in a concentration of from about 10 to 50 mM, more preferably from about 20 to 40 mM, and most preferably about 33 mM.

In practice, the silver ion solution and the reducing agent solution are preferably used in a ratio of about 1:1 and can be premixed before contact with the assay chips or added to the assay chips and then mixed.

In preferred embodiments, silver precipitation on the assay chips is analyzed by an optical detection system. In some preferred embodiments, the optical detection system is included in the housing containing the other system components, while in other embodiments, the optical detection system is located in a separate housing. In some embodiments, the preferred embodiments, the optical detection system comprises an imaging device. In some preferred embodiments, the imaging device is a CCD camera or CMOS device. In some embodiments, the optical detection system comprises a computer programmed to acquire an image via the imaging device, identify and/or quantify light absorbing features (i.e., spots) on the image, correlate the spots to particular capture molecules, controls, or sites on the array, collect the results obtained from the detection device, and carry out a diagnostic and/or quantification of the target analyte(s).

Accordingly, in some embodiments, the detection device is a camera, preferably a CCD or CMOS camera or similar technologies. In some embodiments, the optical detection system further comprises an illuminant source. The light source is preferably visible light having a wavelength similar to the dimension of the metal crystals contained in the precipitate(s) and which is advantageously produced by using a single diode or diodes having the same spectral distribution. The illuminant sources are advantageously regularly spaced around the solid support, each of the sources corresponding to a light spot, which can be automatically switched on simultaneously or successively. The images are preferably obtained either by transparency, by reflection, or by a combination thereof. The system may comprise the use of one camera and one illuminant source, placed above the solid support, the camera and the illuminant source being movable in the three dimensions in space. The system may comprise also the use of two or more cameras oppositely arranged in a plane and placed above the solid support and one or more illuminant sources placed under the solid support. The system may comprise also the use of three or more cameras arranged according to a triangular plane or another regular or irregular pattern and placed above the solid support and one or more illuminant sources placed under the solid support. The system may comprise the use of one camera placed above the solid support, a first illuminant source placed above the solid support and under the camera, a second illuminant source placed under the solid support, the two illuminant sources being placed almost symmetrically according to the position of the solid support.

### B. Use of Detection Reagents in Other Assays

The detection reagents described above, particularly the silver ion solution and reducing agent solution, may also be used in other assay formats where it is desirable to detect gold labeled probes, in particular assays such as Western blots, Northern blots, Southern blots, test strips, microfluidic assays and lateral flow assays.

Thus, in some embodiments, the detection reagents permit the performance of relatively inexpensive, disposable, membrane-based assays or lateral flow assays for the visual identification of the presence (or absence) of an analyte in a liquid sample. Such devices are usually formatted as freestanding dipsticks (e.g., test strips) or as devices having some sort of housing. Typically, an immunoassay device of the present invention can be used with as little as about 200 µl of liquid sample, and detection of an analyte in the sample can (but need not) be complete within 2-5 minutes. In preferred embodiments, no ancillary instrumentation is required to perform such tests, and such devices easily can be used in clinics, laboratories, field locations, and the home even by inexperienced persons.

In some embodiments, immunoassay is an immunochromatographic "sandwich" assay. In general, sandwich immunochromatographic procedures call for mixing the sample that may contain the analyte to be assayed with an antigenic composition or capture reagent as described above. A detector reagent is utilized which is labeled with gold particles. This mixture is then applied to a chromatographic medium containing a band or zone of immobilized antigenic compositions (i.e., the capture reagent). The chromatographic medium often is in the form of a strip that resembles a dipstick. When the complex of antibody and the detector reagent reaches the zone of the immobilized capture antibody on the chromatographic medium, binding occurs and the detector reagent complex is localized at the zone. The reagents of the present invention can then be applied to detect the complex. This technique can be used to obtain quantitative or semi-quantitative results. Examples of sandwich immunoassays performed on test strips are described in U.S. Pat. Nos. 4,168,146 and 4,366,241, each of which is incorporated herein by reference. EP-A 0 125 118 describes such a sandwich type dipstick immunoassay. EP-A 0 282 192 describes a dipstick device for use in competition type assays.

In other embodiments, the assay device of the present invention is a flow through immunoassay device. Flow-through immunoassay devices involve a capture reagent (such as an antigenic composition) bound to a porous membrane or filter to which a liquid sample is added. As the liquid flows through the membrane, target analyte binds to the capture reagent. The addition of sample is followed by (or made concurrent with) addition of detector reagent (e.g., labeled second antibody). Alternatively, the detector reagent may be placed on the membrane in a manner that permits the detector to mix with the sample and thereby label the analyte. The visual detection of detector reagent, which can be accomplished via the reagents described above, provides an indication of the presence of target analyte in the sample. Representative flow-through immunoassay devices are described in U.S. Pat. Nos. 4,246,339; 4,277,560; 4,632,901; 4,812,293; 4,920,046; and 5,279,935; and U.S. Patent Application Publication Nos. 20030049857 and 20040241876, all of which are incorporated by reference in their entirety. In some embodiments, the assay device is a migration assay device. Such devices usually incorporate within them reagents that have been attached to colored labels, thereby permitting visible detection of the assay results without addition of further substances. See, for example, U.S. Pat. No. 4,770,853; PCT Publication No. WO 88/08534 and European Patent No. EP-A 0 299 428, all of which are incorporated by reference in their entirety.

In some embodiments, the reagents of the present invention are used in conjunction with assay lateral flow assay devices. There are a number of commercially available lateral flow type tests and patents disclosing methods for the detection of analytes. See, e.g., U.S. Pat. No. 5,229,073; 5,591,645; 4,168,146; 4,366,241; 4,855,240; 4,861,711; 4,703,017; 5,451,504; 5,451,507; 5,798,273; 6,001,658; and 5,120,643; European Patent No. 0296724; WO 97/06439; and WO 98/36278, all of which are incorporated herein by reference.

The lateral flow assay devices of the present invention include a strip of absorbent or porous material (such as a microporous membrane), which, in some instances, can be made of different substances each joined to the other in zones, which may be abutted and/or overlapped. In some examples, the absorbent strip can be fixed on a supporting non-interactive material (such as nonwoven polyester), for example, to provide increased rigidity to the strip. Zones within each strip may differentially contain the specific binding partner(s) and/or other reagents required for the detection and/or quantification of the particular analyte being tested for. Thus these zones can be viewed as functional sectors or functional regions within the test device.

In some embodiments, a fluid sample (or a sample suspended in a fluid) is introduced to the strip at the proximal end of the strip, for instance by dipping or spotting. A sample is collected or obtained using methods well known to those skilled in the art. The sample containing the analyte to be detected may be obtained from any biological source. Examples of biological sources include blood serum, blood plasma, urine, spinal fluid, saliva, fermentation fluid, lymph fluid, tissue culture fluid and ascites fluid of a human or animal. The sample may be diluted, purified, concentrated, filtered, dissolved, suspended or otherwise manipulated prior to immunoassay to optimize the immunoassay results. The fluid migrates distally through all the functional regions of the strip. The final distribution of the fluid in the individual functional regions depends on the adsorptive capacity and the dimensions of the materials used.

In some embodiments, the assay devices include a detector reagent. The detector reagent provides a means to detect the formation of a complex between an analyte (such as a target antibody or antibodies) and a capture reagent (such as an antigenic composition as described above). A detector may be integrated into an immunoassay device (for example included in a conjugate pad, as described below), or may be applied to the device from an external source. In preferred embodiments, the detector reagent is labeled with gold and is detected with the reagents described above.

The flow-through devices of the present invention comprise a capture reagent (e.g., antigenic composition as described above) immobilized on a solid support such as a microtiter plate or a membrane (such as, nitrocellulose, nylon, or PVDF). Characteristics of useful membrane have been previously described; however, it is useful to note that in a flow-through assay capillary rise is not a particularly important feature of a membrane as the sample moves vertically through the membrane rather than across it as in a lateral flow assay. In a simple representative format, the membrane of a flow-through device is placed in functional or physical contact with an absorbent layer (see, e.g., description of "absorbent pad" below), which acts as a reservoir to draw a fluid sample through the membrane. Optionally, following immobilization of a capture reagent, any remaining protein-binding sites on the membrane can be blocked (either before or concurrent with sample administration) to minimize nonspecific interactions.

### C. Kits

In some embodiments, the present invention provides kits for detection of gold labeled probes. In some preferred embodiment, the kits comprise a first container with silver ion solution and a second container with reducing agent solution. The kits may optionally contain one or more additional containers with wash solution, gold-labelled probes and control reagents.

### CLAUSES:

Clause 1. A system for automated detection of gold-labelled probes on a microarray comprising:
   a dispensing platform configured to receive a solid support having thereon an array comprising discrete regions with a density of at least 20 of the discrete regions per cm², each of the discrete regions being fixed with one species of capture molecule specific for an analyte,
   a plurality of reservoirs separately containing a plurality reagents comprising at least a silver ion solution, a reducing agent solution, one or more gold-labelled probes specific for the analyte, one or more test samples and one or more wash solutions,
   a reagent dispensing system coupled to the plurality of reservoirs and configured to dispense the plurality of reagents onto a solid support disposed on the dispensing platform,
   a control system coupled with the dispensing system to allow ordered dispensation of the reagents onto a solid support disposed on the dispensing platform.
Clause 2. The system of clause 1, further comprising a mixing reservoir in fluid communication with the reservoirs contains the silver ion solution and the reducing agent solution.
Clause 3. The system of clause 1, wherein the dispensing platform is configured for controlled agitation of the solid support.
Clause 4. The system of clause 1, wherein the platform is heated.
Clause 5. The system of clause 1, further comprising an optical detection system.
Clause 6. The system of clause 5, wherein the optical detection system comprises an imaging device.
Clause 7. The system of clause 6, wherein the imaging device is a CCD camera.
Clause 8. The system of clause 1, wherein the dispensing platform, plurality of reservoirs, reagent dispensing system and control system are in a housing.
Clause 9. The system of clause 1, wherein the dispensing platform is moveable between at least two positions and the movement is controlled by the control system.
Clause 10. The system of clause 1, wherein the reagent dispensing system is moveable between at least two positions and the movement is controlled by the control system. Clause 11. The system of clause 1, wherein the silver ion solution comprises a solution of AgNO₃.
Clause 12. The system of clause 1, wherein the molarity of the silver nitrate in the solution is from about 1 to about 50 mM.
Clause 13. The system of clause 1, wherein the reducing agent solution comprising a reducing agent selected from the group consisting of p-aminophenol, phenidone (1-phenyl-3-pyrazolidinone) and hydroquinone.
Clause 14. The system of clause 13, wherein the molarity of the reducing agent in the reducing agent solution is from about 0.5 to about 10 mM.
Clause 15. The system of clause 14, wherein the solution further comprises sodium citrate at a concentration from about 50 to about 175 mM.
Clause 16. The system of clause 14, wherein the solution further comprises citric acid at a concentration from about 50 to about 175 mM.
Clause 17. The system of clause 13, wherein the pH of the reducing agent solution is from about 4.0 to about 5.0.
Clause 18. An automated process for enhancing detection of gold-labelled probes on a microarray comprising:
   providing a solid support having thereon an array comprising discrete regions with a density of at least 20 of the discrete regions per cm², each of the discrete regions being fixed with one species of capture molecule bound to an analyte labelled with a gold probe, a first container comprising a silver ion solution and a second container comprising a reducing agent solution;
   dispensing the silver ion solution and the reducing agent solution into a third container;
   premixing the silver ion solution and the reducing agent solution to provide a premixed silver enhancement solution;
   dispensing the premixed silver enhancement solution onto the solid support; and
   incubating the solid support under conditions such that the silver ions nucleate on the gold particles,
   wherein said array comprises one or more discrete control regions in at least two separate quadrants on said array and the amount of silver precipitation at each of said discrete control regions varies by less than 10%.
19. The process of clause 18, wherein the silver ion solution comprises a solution of AgNO₃.
Clause 20. The process of clause 18, wherein the molarity of the silver nitrate in the solution is from about 1 to about 50 mM.
Clause 21. The process of clause 18, wherein the reducing agent solution comprising a reducing agent selected from the group consisting of phenidone (1-phenyl-3-pyrazolidinone) and hydroquinone.
Clause 22. The process of clause 22, wherein the molarity of the reducing agent in the reducing agent solution is from about 0.5 to about 10 mM.
Clause 23. The process of clause 21, wherein the solution further comprises sodium citrate at a concentration from about 50 to about 175 mM.
Clause 24. The process of clause 21, wherein the solution further comprises citric acid at a concentration from about 50 to about 175 mM.
Clause 25. The process of clause 21, wherein the pH of the reducing agent solution is from about 4.0 to about 5.0.
Clause 26. The process of clause 18, wherein the process is implemented by the automated system of claim 1.
Clause 27. An automated process for enhancing detection of gold-labelled probes on a microarray comprising:
   providing a solid support having thereon an array comprising discrete regions with a density of at least 20 of the discrete regions per cm², each of the discrete regions being fixed with one species of capture molecule bound to an analyte labelled with a gold probe, a first container comprising a silver ion solution and a second container comprising a reducing agent solution;
   dispensing the silver ion solution and the reducing agent solution onto the solid surface;
   agitating the solid support to mix the silver ion solution and the reducing agent solution; and
   incubating the solid support under conditions such that the silver ions nucleate on the gold particles,
   wherein said array comprises one or more discrete control regions in at least two separate quadrants on said array and the amount of silver precipitation at each of said discrete control regions varies by less than 10%.
Clause 28. The process of clause 27, wherein the silver ion solution comprises a solution of AgNO₃.
Clause 29. The process of clause 27, wherein the molarity of the silver nitrate in the solution is from about 1 to about 50 mM.
Clause 30. The process of clause 27, wherein the reducing agent solution comprising a reducing agent selected from the group consisting of p-aminophenol, phenidone (1-phenyl-3-pyrazolidinone) and hydroquinone.
Clause 31. The process of clause 30, wherein the molarity of the reducing agent in the reducing agent solution is from about 0.5 to about 10 mM.
Clause 32. The process of clause 30, wherein the solution further comprises sodium citrate at a concentration from about 50 to about 175 mM.
Clause 33. The process of clause 30, wherein the solution further comprises citric acid at a concentration from about 50 to about 175 mM.
Clause 34. The process of clause 30, wherein the pH of the reducing agent solution is from about 4.0 to about 5.0.
Clause 35. The process of clause 27, wherein the process is implemented by the automated system of claim 1.

## Claims

1. An automated process for enhancing detection of gold-labelled probes on a microarray comprising:
providing a solid support having thereon an array comprising discrete regions with a density of at least 20 of the discrete regions per cm², each of the discrete regions being fixed with an antigen capture molecule bound to an analyte labelled with a gold probe, a first container comprising a silver ion solution and a second container comprising a reducing agent solution;
dispensing the silver ion solution and the reducing agent solution into a third container;
premixing the silver ion solution and the reducing agent solution to provide a premixed silver enhancement solution;
dispensing the premixed silver enhancement solution onto the solid support; and
incubating the solid support under conditions such that the silver ions nucleate on the gold particles,
wherein said array comprises one or more discrete control regions in at least two separate quadrants on said array and the amount of silver precipitation at each of said discrete control regions varies by less than 10%,
wherein the molarity of the reducing agent in the reducing agent solution is from about 0.5 to about 10 mM,
wherein the reducing agent solution further comprises sodium citrate at a concentration from about 50 to about 175 mM and/or wherein the solution further comprises citric acid at a concentration from about 50 to about 175 mM,
wherein the reducing agent solution further comprises 4-hydroxy-D-phenylglycine in a concentration from 10 to 50 mM, and
wherein the pH of the reducing agent solution is from about 4.0 to about 5.0.

2. The process of claim 1, wherein the silver ion solution comprises a solution of AgNO₃ preferably wherein the molarity of the silver nitrate in the solution is from about 1 to about 50 mM.

3. The process of claim 1, wherein the process is implemented by a system for automated detection of gold-labelled probes on a microarray comprising:
a dispensing platform configured to receive a solid support having thereon an array comprising discrete regions with a density of at least 20 of the discrete regions per cm², each of the discrete regions being fixed with one species of capture molecule specific for an analyte,
a plurality of reservoirs separately containing a plurality reagents comprising at least a silver ion solution, a reducing agent solution, one or more gold-labelled probes specific for the analyte, one or more test samples and one or more wash solutions,
a reagent dispensing system coupled to the plurality of reservoirs and configured to dispense the plurality of reagents onto a solid support disposed on the dispensing platform,
a control system coupled with the dispensing system to allow ordered dispensation of the reagents onto a solid support disposed on the dispensing platform,
wherein the molarity of the reducing agent in the reducing agent solution is from about 0.5 to about 10 mM,
wherein the reducing agent solution further comprises sodium citrate at a concentration from about 50 to about 175 mM and/or wherein the solution further comprises citric acid at a concentration from about 50 to about 175 mM,
wherein the reducing agent solution further comprises sodium thiosulfite at a concentration from 0.1 to 1 mM and 4-hydroxy-D-phenylglycine in a concentration from 10 to 50 mM, and
wherein the pH of the reducing agent solution is from about 4.0 to about 5.0.

4. An automated process for enhancing detection of gold-labelled probes on a microarray comprising:
providing a solid support having thereon an array comprising discrete regions with a density of at least 20 of the discrete regions per cm², each of the discrete regions being fixed with an antigen capture molecule bound to an analyte labelled with a gold probe, a first container comprising a silver ion solution and a second container comprising a reducing agent solution;
dispensing the silver ion solution and the reducing agent solution onto the solid surface;
agitating the solid support to mix the silver ion solution and the reducing agent solution; and
incubating the solid support under conditions such that the silver ions nucleate on the gold particles,
wherein said array comprises one or more discrete control regions in at least two separate quadrants on said array and the amount of silver precipitation at each of said discrete control regions varies by less than 10%,
wherein the molarity of the reducing agent in the reducing agent solution is from about 0.5 to about 10 mM,
wherein the reducing agent solution further comprises sodium citrate at a concentration from about 50 to about 175 mM and/or wherein the solution further comprises citric acid at a concentration from about 50 to about 175 mM,
wherein the reducing agent solution further comprises 4-hydroxy-D-phenylglycine in a concentration from 10 to 50 mM, and
wherein the pH of the reducing agent solution is from about 4.0 to about 5.0.

5. The process of claim 4, wherein the silver ion solution comprises a solution of AgNO₃ preferably wherein the molarity of the silver nitrate in the solution is from about 1 ato about 50 mM.

6. The process of claim 5, wherein the process is implemented by a system for automated detection of gold-labelled probes on a microarray comprising:
a dispensing platform configured to receive a solid support having thereon an array comprising discrete regions with a density of at least 20 of the discrete regions per cm², each of the discrete regions being fixed with one species of capture molecule specific for an analyte,
a plurality of reservoirs separately containing a plurality reagents comprising at least a silver ion solution, a reducing agent solution, one or more gold-labelled probes specific for the analyte, one or more test samples and one or more wash solutions,
a reagent dispensing system coupled to the plurality of reservoirs and configured to dispense the plurality of reagents onto a solid support disposed on the dispensing platform,
a control system coupled with the dispensing system to allow ordered dispensation of the reagents onto a solid support disposed on the dispensing platform,
wherein the molarity of the reducing agent in the reducing agent solution is from about 0.5 to about 10 mM,
wherein the reducing agent solution further comprises sodium citrate at a concentration from about 50 to about 175 mM and/or wherein the solution further comprises citric acid at a concentration from about 50 to about 175 mM,
wherein the reducing agent solution further comprises sodium thiosulfite at a concentration from 0.1 to 1 mM and 4-hydroxy-D-phenylglycine in a concentration from 10 to 50 mM, and wherein the pH of the reducing agent solution is from about 4.0 to about 5.0.
